# EUROPÄISCHE PATENTSCHRIFT

(11) **EP 1 165 596 B1**
(45) Veröffentlichungstag und Bekanntmachung des Hinweises auf die Patenterteilung: **19.06.2002**
(21) Anmeldenummer: 00922533.5
(22) Anmeldetag: 22.03.2000
(51) Int. Cl.: C07J 53/00, A61K 31/565, C07J 71/00

(54) **14,15-ALPHA-METHYLEN-EQUILENINDERIVATE, VERFAHREN ZU DEREN HERSTELLUNG UND DIESE ENTHALTENDE ARZNEIMITTEL**
14,15-ALPHA-METHYLENE EQUILENINE DERIVATIVES, METHODS FOR PRODUCING THE SAME AND MEDICAMENTS CONTAINING THEM
DERIVES DE 14,15-ALPHA-METHYLENE EQUILENINE, PROCEDES PERMETTANT DE LES PREPARER ET MEDICAMENTS LES CONTENANT

(30) Priorität: 30.03.1999 DE 19915576
(43) Veröffentlichungstag der Anmeldung: 02.01.2002
(73) Patentinhaber: Jenapharm GmbH & Co. KG, 07745 Jena (DE)
(72) Erfinder: SCHWARZ, Sigfrid, D-07743 Jena (DE); THIEME, Ina, D-07616 Graitschen (DE); UNDEUTSCH, Bernd, D-07745 Jena (DE); KAUFMANN, Günter, D-07743 Jena (DE); RÖMER, Wolfgang, D-07749 Jena (DE)
(74) Vertreter: Cramer, Eva-Maria
(86) Internationale Anmeldenummer: EP0002513
(87) Internationale Veröffentlichungsnummer: WO0059922

(56) Entgegenhaltungen:
- EP-A- 0 753 300
- WO-A-95/13076
- WO-A-98/25626
- KUENZER, H. ET AL: "A concise total synthesis of C(14)-C(15) methylene-bridged equilenin derivatives" TETRAHEDRON LETT. (1994), 35(15), 2329-30 , XP002143082
- ROMER W ET AL: "Novel ?scavestrogens ? and their radical scavenging effects, iron-chelating, and total antioxidative activities: DELTA-dehydro derivatives of 17alpha-estradiol and 17beta-estradiol" STEROIDS: STRUCTURE, FUNCTION, AND REGULATION,US,ELSEVIER SCIENCE PUBLISHERS, NEW YORK, NY, Bd. 62, Nr. 3, 1. März 1997 (1997-03-01), Seiten 304-310, XP004057109 ISSN: 0039-128X
- ROEMER W ET AL: "SCAVESTROGEN SULFAMATES: CORRELATION BETWEEN ESTRONE SULFATASE INHIBITING AND ANTIOXIDANT EFFECTS" CANADIAN JOURNAL OF PHYSIOLOGY AND PHARMACOLOGY,CA,OTTAWA, ONT, Bd. 76, Nr. 2, 1998, Seiten 99-109, XP000852556
- N. KOIZUMI ET AL: "Antiandrogen. IV. C-17-Spiro 2-Oxasteroids" CHEMICAL AND PHARMACEUTICAL BULLETIN, Bd. 44, Nr. 11, November 1996 (1996-11), Seiten 2162-2164, XP002143083 TOKYO JP
- K. SHIBATA ET AL: "Antiandrogen. I. 2-Azapregnane and 2-Oxapregnane Steroids" CHEMICAL AND PHARMACEUTICAL BULLETIN., Bd. 40, Nr. 4, April 1992 (1992-04), Seiten 935-941, XP002143084 PHARMACEUTICAL SOCIETY OF JAPAN. TOKYO., JP ISSN: 0009-2363

## Beschreibung

Die vorliegende Erfindung betrifft neue Equileninderivate, Verfahren zu deren Herstellung sowie diese Verbindungen enthaltende Arzneimittel.

Equilenin selbst ist ein estrogenes Steroid, welches aus dem Ham trächtiger Stuten gewinnbar ist.
Die erfindungsgemäßen neuen Equileninderivate weisen eine Fluor- oder eine Sauerstoffunktion am C-Atom 11 und eine α-ständige Methylenbrücke zwischen den C-Atomen 14 und 15 auf. Equileninderivate mit einer Sauerstoffunktion am C-Atom 11 sind bekannt. So wurde racemischer 11-Oxo-equileninmethylether durch Totalsynthese erhalten (Tetrahedron Lett. 2763 (1967); Aust. J. Chem. 23, 547 (1970); J. Org. Chem. 39, 2193 (1974)). Auf totalsynthetischem Weg war auch racemische 11-Oxo-3-methoxy-estra1,3,5(10),6,8,14-hexaen-17β-yl-carbonsäure zugänglich (Tetrahedron Lett. 479 (1968)). 14α,17α-überbrückte Equileninderivate mit einer 11-Sauerstoffunktion wurden partialsynthetisch erhalten. Die Einführung der 11-Sauerstoffunktion in das Molekül erfolgte mit Cer-IV-ammoniumnitrat (Tetrahedron Lett. 35, 8599 (1994)). Equileninderivate mit einer α- oder β-ständigen Methylenbrücke zwischen den C-Atomen 14 und 15 wurden ebenfalls partialsynthetisch hergestellt, wobei der B-Ring mit Dichlordicyanobenzochinon (DDQ) dehydriert wurde (Tetrahedron Lett. 35, 2329 (1994)).

Verwandte Steroide mit antioxidativen Eigenschaften werden in EP-A-753 300 offenbart.

Aufgabe der vorliegenden Erfindung ist es, neue Equileninderivate sowie ein Verfahren zu deren Herstellung zur Verfügung zu stellen.
Erfindungsgemäß wird diese Aufgabe dadurch gelöst, daß Equileninderivate der allgemeinen Formel I 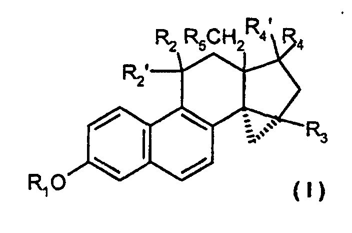 geschaffen werden, in der
R₁ ein Wasserstoffatom, eine C₁-C₅-Alkyl- oder eine C₁-C₅-Acylgruppe oder eine Benzoylgruppe bedeutet,
R₂ ein Wasserstoffatom und R₂' ein Fluoratom, eine Hydroxygruppe oder eine C₁-C₅-Acyloxygruppe darstellt oder R₂ und R₂' zusammen eine Oxogruppe darstellen,
R₃ ein Wasserstoffatom oder eine Methylgruppe darstellt,
R₄ ein Wasserstoffatom und R₄' eine Hydroxygruppe oder eine C₁-C₁₁-Acyloxygruppe darstellt oder R₄ und R₄' zusammen eine Oxogruppe, eine Methylengruppe, eine Halogenmethylengruppe oder eine Dihalogenmethylengruppe darstellen und
R₅ ein Wasserstoffatom oder eine Methylgruppe ist.
Erfindungsgemäß bevorzugt ist es, wenn R₅ ein Wasserstoffatom ist.

Erfindungsgemäß besonders bevorzugte Equileninderivate sind beispielsweise:
1) 14α,15α-Methylen-estra-1,3,5(10),6,8-pentaen-3,11β,17β-triol,
2) 11β,17β-Dihydroxy-14α,15α-methylen-estra-1,3,5(10),6,8-pentaen-3-yl-benzoat,
3) 11β,17β-Dihydroxy-14α,15α-methylen-estra-1,3,5(10),6,8-pentaen-3-ylpropionat,
4) 3,11β-Dihydroxy-14α,15α-methylen-estra-1,3,5(10),6,8-pentaen-17β-yl-decanoat,
5) 3,11β-Dihydroxy-14α,15α-methylen-estra-1,3,5(10),6,8-pentaen-17-on,
6) 3-Methoxy-14α,15α-methylen-estra-1,3,5(10),6,8-pentaen-11α,17β-diyl-diacetat,
7) 15β-Methyl-14α,15α-methylen-estra-1,3,5(10),6,8-pentaen-3,11β,17β-triol,
8) 11β-Fluor-14α,15α-methylen-estra-1,3,5(10),6,8-pentaen-3,17β-diol,
9) 3,17β-Dihydroxy-14α,15α-methylen-1,3,5(10),6,8-pentaen-11-on,
10) 3-Methoxy-14α,15α-methylen-estra-1,3,5(10),6,8-pentaen-11α,17α-diyl-diacetat,
11) 3-Methoxy-14α,15α-methylen-11-oxo-estra-1,3,5(10),6,8-pentaen-17α-yl-acetat,
12) 11β-Hydroxy-17,17-difluormethylen-14α,15α-methylen-estra-1,3,5(10),6,8-pentaen-3-yl-benzoat und
13) 14α,15α,17,17-Bis-methylen-estra-1,3,5(10),6,8-pentaen-3,11α-diol.

Unter "C₁-C₅-Alkyl" wird im Sinne der vorliegenden Erfindung ein verzweigter oder geradkettiger Alkylrest verstanden. Als Beispiele seien eine Methyl-, Ethyl-, n-Propyl-, i-Propyl-, n-Butyl-, i-Butyl- oder tert.-Butyl-, n-Pentyl- oder i-Pentylgruppe genannt. Unter dem Begriff "C₁₋₅- bzw. C₁₋₁₁-Acyl-" wird im Sinne der vorliegenden Anmeldung ein Rest mit 1 bis 5 bzw. 1 bis 11 Kohlenstoffatomen der geradkettigen oder verzweigten Alkancarbonsäuren, wie beispielsweise der Ameisensäure, Essigsäure, Propionsäure, Butansäure, iso-Butansäure, Heptansäure oder Undecansäure, verstanden.
Unter dem Begriff "Halogen" wird im Sinne der vorliegenden Erfindung ein Fluor-, Chlor-, Brom- oder lodatom verstanden.
Die erfindungsgemäßen Equileninderivate sind neu. Sie wurden bisher weder hergestellt noch wurden ihre Eigenschaften beschrieben. Die erfindungsgemäßen Equileninderivate weisen antioxidative Aktivität bei geringer systemischer hormoneller Wirkung auf. Die antioxidative Wirkung wurde unter anderem durch Hemmung der Eisen-II-katalysierten Lipidperoxidation in synaptosomalen Membranfraktionen der Ratte, Hemmung der Kupfer-II-sulfat-induzierten LDL-Cholesterol-Oxidation und Hemmung der Xanthinoxidase sowie verschiedener anderer Monooxygenasen bestimmt. Auf systemische estrogene Wirkung wurde im Allen-Doisy-Test an der Ratte geprüft. Das gemessene Wirkungsspektrum der erfindungsgemäßen Equileninderivate bietet Möglichkeiten für eine therapeutische Anwendung in all jenen Fällen, in denen Sauerstoffradikale in ursächlichem Zusammenhang mit Erkrankungen von Organen oder Geweben stehen, wie beispielsweise bei Hirn- und Wirbelsäulenverletzungen, Schockzuständen, Emphysemen, ARDS, Alterungsprozessen, Gewebeschädigungen nach Myokardinfarkt, Vergiftungs- und Verstrahlungsschäden, Verbrennungen und transplantationsbedingten Immunreaktionen, wie Organschäden in der Reperfusionphase nach Transplantationen, beim spinalen Trauma, bei Schlaganfall, Arteriosklerose, Ischämie, chronisch-degenerativen Erkrankungen des ZNS, seniler Demenz vom Alzheimer-Typ (SDAT), Asthma, muskulärer Dystrophie und degenerativen neurologischen Krankheiten u. a. in Form von ZNS-Intoxikations- bzw. Degenerationszuständen. Ein bevorzugtes Anwendungsfeld ist hierbei die Geroprophylaxe bei Frauen und - bedingt durch die gering feminisierende Wirkung der Verbindungen - auch bei Männern.

Hierbei können die erfindungsgemäßen Verbindungen sowohl oral als auch parenteral verabreicht werden. Bei der oralen Applikation sind Prodrugs in Form von Carbonsäureestem besonders vorteilhaft, da sie langanhaltend gleichbleibende Wirkstoffspiegel ermöglichen.

Ein weiterer Gegenstand der vorliegenden Erfindung ist ein Verfahren zur Herstellung der erfindungsgemäßen Equileninderivate der allgemeinen Formel I 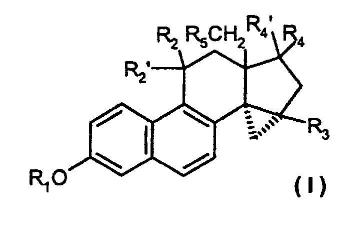 worin R₁, R₂, R₂', R₃, R₄, R₄' und R₅ die oben angegebene Bedeutung haben, indem man eine Verbindung der allgemeinen Formel II 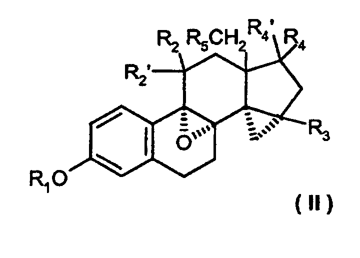 worin R₁, R₂, R₂', R₃, R₄, R₄' und R₅ die oben angegebene Bedeutung haben, mit Diphosphortetraiodid in Gegenwart von Pyridin zur Reaktion bringt und die so erhaltenen Verbindungen in an sich bekannter Weise zu den Verbindungen der allgemeinen Formel I umsetzt.
Es ist bekannt, daß Diphosphortetraiodid mit Epoxiden und Alkoholen reagiert. So können Epoxide mit Diphosphortetraiodid zu Olefinen reduziert werden (Synthesis 905 (1978); Nouv. J. Chem. 3, 745 (1979)). Alkohole reagieren mit Diphosphortetraiodid unter Bildung von lodiden (Tetrahedron Letters 1801(1979); J. C. S. Chem. Commun. 229 (1983)) oder unter Eliminierung zu Olefinen (Helv. Chim. Acta 11,106 (1928)) oder zu Kumulenen (Ber. 71, 1899 (1938); ibid. 85, 386 (1952); ibid. 87, 598 (1954); J. C. S. Chem. Commun. 885 (1975)). Das erfindungsgemäße Verfahren zeichnet sich dadurch aus, daß die Einwirkung von Diphosphortetraiodid auf Verbindungen der allgemeinen Formel II zur Eliminierung der 8,9-Oxidogruppe und gleichzeitig zur Einführung einer zusätzlichen Doppelbindung zwischen den C-Atomen 6 und 7 führt. Damit wird die Herstellung der erfindungsgemäßen Equileninderivate der allgemeinen Formel I aus Verbindungen der allgemeinen Formel II in einem Schritt möglich und eine zusätzliche Reaktionsstufe zur Einführung der 6,7-Doppelbindung (Tetrahedron Letters 35, 2329 (1994)) umgangen. Das erfindungsgemäße Verfahren zeichnet sich weiterhin dadurch aus, daß - sofern man Verbindungen der allgemeinen Formel II, in der R₂ Wasserstoff und R₂'eine Hydroxygruppe bedeutet, zum Einsatz bringt - weder eine Eliminierung der ungeschützten Hydroxygruppe zum betreffenden Olefin noch eine Substitution der Hydroxygruppe durch lod erfolgt. Der Verlauf und die hohe Selektivität des erfindungsgemäßen Verfahrens sind überraschend und waren vom Fachmann nicht vorhersehbar.

Verbindungen der allgemeinen Formel II sind aus Verbindungen der allgemeinen Formel III, in der R₁ und R₃ bis R₅, die unter Verbindung II genannten Bedeutungen zukommen, zugänglich, indem man diese mit überschüssiger Peroxycarbonsäure behandelt.

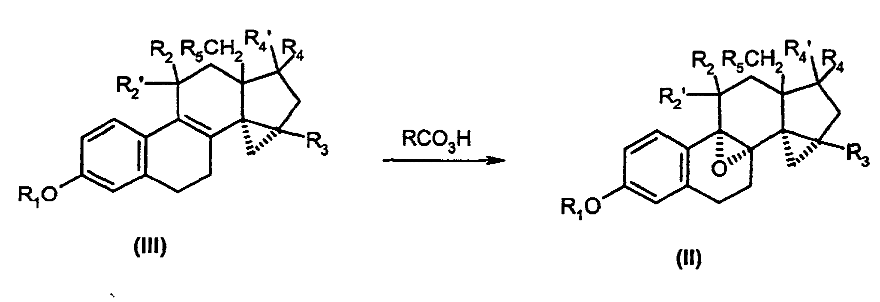

Die erfindungsgemäß erhaltenen Equileninderivate können gegebenenfalls nach an sich bekannten Methoden weiter strukturell abgewandelt werden. So ist es beispielsweise möglich, Verbindungen der allgemeinen Formel I, in der R₂' eine α-Hydroxygruppe und R₂ einen β-Wasserstoff bedeutet, in an sich bekannter Weise einer Oxidation mit aktiviertem Dimethylsulfoxid zu unterziehen, wobei die entsprechenden 11-Oxoverbindungen entstehen, deren Reduktion mit einem komplexen Metallhydrid die entsprechenden 11β-Hydroxyderivate ergibt. Alternativ führt die Reaktion von Verbindungen der allgemeinen Formel I, in der R₂' eine α-Hydroxygruppe und R₂ einen β-Wasserstoff bedeutet, mit Diethylamino-Schwefeltrifluorid (DAST) zu Verbindungen mit einer 11β-Fluorgruppe. Verbindungen der allgemeinen Formel I, in der R₄' einen C₁-C₅-Alkylrest darstellt, sind in an sich bekannter Weise mit Bortribromid oder Diisobutylaluminiumhydrid in die freien Phenole überführbar. Verbindungen der allgemeinen Formel I, in der R₄' eine α-Hydroxygruppe und R₄ einen β-Wasserstoff darstellt, können in an sich bekannter Weise mit aktiviertem Dimethylsulfoxid oxidiert werden, wobei die entsprechenden 17-Oxosteroide entstehen, deren Reduktion mit Boran oder Oxazaborolidinen die entsprechenden 17β-Hydroxyverbindungen ergibt.

Die Cyclopropano-Steroide der allgemeinen Formel II 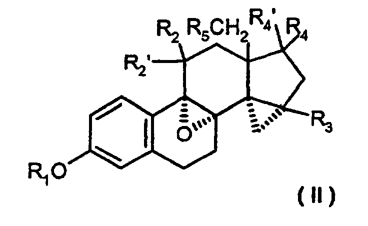 in der
R₁ ein Wasserstoffatom, eine C₁-C₅-Alkyl- oder eine C₁-C₅-Acylgruppe oder eine Benzoylgruppe bedeutet,
R₂ ein Wasserstoffatom und R₂'ein Fluoratom, eine Hydroxygruppe oder eine C₁-C₅₋ Acyloxygruppe darstellt oder R₂ und R₂' zusammen eine Oxogruppe darstellen,
R₃ ein Wasserstoffatom oder eine Methylgruppe darstellt,
R₄ ein Wasserstoffatom und R₄' eine Hydroxygruppe oder eine C₁-C₁₁-Acyloxygruppe darstellt oder R₄ und R₄' zusammen eine Oxogruppe, eine Methylengruppe, eine Halogenmethylengruppe oder eine Dihalogenmethylengruppe darstellen und
R₅ ein Wasserstoffatom oder eine Methylgruppe ist, sind neu und bisher nicht beschrieben worden.

Besonders bevorzugt sind hierbei die folgenden beispielhaft genannten Cyclopropano-Steroide.
1. 11α-Hydroxy-3-methoxy-14α,15α-methylen-8α,9α-oxido-estra-1,3,5(10)-trien-17α-yl-acetat,
2. 3-Methoxy-14α,15α-methylen-8α,9α-oxido-estra-1,3,5(10)-trien-11α,17α-diyl-diacetat und
3. 3-Methoxy-11α-hydroxy-8α,9α-oxido-14α,15α-methylen-estra-1,3,5(10)-trien-17β-yl-acetat.

Diese Verbindungen stellen neue Zwischenprodukte zu den erfindungsgemäßen Equileninderivaten dar und sind somit ein weiterer Gegenstand der vorliegenden Erfindung.

Gegenstand der vorliegenden Erfindung sind auch Arzneimittel zur oralen, transdermalen, rektalen, subcutanen, intravenösen oder intramuskulären Applikation, die neben üblichen Träger- und Verdünnungsmitteln eine Verbindung der allgemeinen Formel I als Wirkstoff enthalten.

Die Arzneimittel der Erfindung werden mit den üblichen festen oder flüssigen Trägerstoffen oder Verdünnungsmitteln und den üblicherweise verwendeten pharrnazeutisch-technischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist Solche Darreichungsformen sind beispielsweise Tabletten, Filmtabletten, Dragees, Kapseln, Pillen, Pulver, Lösungen oder Suspensionen oder Depofformen.

Selbstverständlich kommen auch parenterale Zubereitungen wie Injektionslösungen in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln wie Dextrose, Zukker, Sorbit, Mannit, Polyvinylpyrrolidon, Sprengmitteln wie Maisstärke oder Alginsäure, Bindemitteln wie Stärke oder Gelantine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäßen Wirkstoff können zusätzlich geschmacksverbessemde Mittel wie Saccharin, Cyclamat oder Zucker sowie z. B. Aromastoffe wie Vanillin oder Orangenextrakt enthalten. Sie können außerdem Suspendierhilfsstoffe wie Natriumcarboxymethylcellulose oder Konservierungsstoffe wie p-Hydroxybenzoate enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt.
Geeignete Suppositorien lassen sich beispielsweise durch Vermischen mit dafür vorgesehenen Trägermitteln wie Neutralfetten oder Polyethylenglykol bzw. deren Derivaten herstellen.
Transdermale Applikationsformen können beispielsweise aus wirkstoffhaltigen Pflastern bestehen. Derartige Systeme sind bekannt.
Die nachfolgenden Beispiele erläutern die Erfindung.

### Beispiel 1

### 11α-Hydroxy-3-methoxy-14α,15α-methylen-8α,9α-oxido-estra-1,3,5(10)-trien-17α-yl-acetat aus 3-Methoxy-14α,15α-methylen-estra-1,3,5(10),8-tetraen-17α-yl-acetat

Eine Lösung aus dem Steroid-Tetraen (3,5 g) in Dichlormethan (120 ml) wird bei Raumtemperatur mit Peroxyessigsäure (32 %ig, 5,5 ml) versetzt. Man läßt die Reaktionsmischung über Nacht bei Raumtemperatur stehen. Danach behandelt man die Lösung nacheinander mit wäßriger Natriumthiosulfatlösung (20 %ig), gesättigter wäßriger Natriumhydrogencarbonatlösung und mit Wasser. Die organische Phase wird über Magnesiumsulfat getrocknet und im Vakuum eingeengt. Den Rückstand unterwirft man einer Flashchromatographie an Kieselgel (Eluent: Cyclohexan: Ethylacetat 3:2 v/v). Umkristallisation aus Aceton/n-Hexan ergibt die Titelverbindung;
Fp. 159-162,5 °C. ¹H-NMR (CDCl₃/TMS): 7.80 (d, J = 8,8 Hz, H-1), 6,79 (dd, J = 8,8, 2,8 Hz, H-2), 6,65 (d, J = 2,8 Hz, H-4), 4,93 (q, J = 7,9 Hz, H-11), 4,78 (d, J = 5,9 Hz, H-17), 3,80 (s, -OCH3), 2,03 (s, -OOC-CH₃), 1,11 (dd, J = 5,4, 3,2 Hz, 14,15-CH₂-), 0,88 (s, H-18), 0,69 (ddd, J = 6,6, 5,4, 1,7 Hz, 14,15-CH₂-). MS (m/z): 354 (M⁺), 336, 294, 277, 261.

### Beispiel 2

### 3-Methoxy-14α,15α-methylen-8α,9α-oxido-estra-1,3,5(10)-trien-11α,17α-diyl-diacetat aus 11α-Hydroxy-3-methoxy-14α,15α-methylen-8α,9α-oxido-estra-1,3,5(10)-trien-17α-yl-acetat

Zu einer Lösung des 11α-Hydroxysteroids (0,4 g) in Pyridin (4 ml) gibt man bei Raumtemperatur Acetanydrid (4 ml) und Dimethylaminopyridin (0,04 g). Man rührt das Gemisch 3 Stunden bei Raumtemperatur und gießt dann in Eiswasser. Der entstandene Niederschlag wird abfiltriert, mit Wasser neutral gewaschen und an der Luft getrocknet. Durch Flashchromatographie an Kieselgel (Eluent: Cyclohexan:Ethylacetat 7:3 v/v) erhält man die Titelverbindung.
Fp. 151-154 °C. ¹H-NMR (CDCl₃/TMS) : 7,80 (d, J = 8,8 Hz, H-1), 6,79 (dd, J = 8,8, 2,8 Hz, H-2), 6,65 (d, J = 2,8 Hz, H-4), 4,93 (q, J = 7,9 Hz, H-11), 4,78 (d, J = 5,9 Hz, H-17), 3,80 (s, -OCH3), 2,03 (s, -OOC-CH₃), 1,11 (dd, J = 5,4, 3,2 Hz, 14,15-CH₂-), 0,88 (s, H-18), 0,69 (ddd, J = 6,6, 5,4, 1,7 Hz, 14,15-CH₂-). MS (m/z): 354 (M⁺) 336, 294, 277, 261.

### Beispiel 3

### 3-Methoxy-14α,15α-methylen-estra-1,3,5(10),6,8-pentaen-11α,17α-diyl-diacetat aus 3-Methoxy-14α,15α-methylen-8α,9α-oxido-estra-1,3,5(10)-trien-11α,17α-diyl-diacetat

Zu einer gerührten Suspension von Diphosphortetraiodid (0,14 g) in Chloroform (2,4 ml) wird unter Argonschutz und bei Raumtemperatur eine Lösung, bestehend aus dem Steroiddiacetat (0,1 g), Chloroform (2,4 ml) und Pyridin (0,24 ml) zugetropft. Anschließend wird 13 Stunden unter Rühren am Rückfluß zum Sieden erhitzt. Man gibt Wasser zu, trennt die organische Phase ab und extrahiert die wäßrige Phase mit Chloroform erschöpfend nach. Die vereinigten organischen Phasen werden nacheinander mit Salzsäure (1 N), Wasser, gesättigter wäßriger Natriumhydrogencarbonatlösung und gesättigter wäßriger Natriumchloridlösung gewaschen, über Magnesiumsulfat getrocknet und im Vakuum eingeengt. Den Rückstand unterwirft man der Flashchromatographie, wobei die Titelverbindung erhalten wird.
¹H-NMR (CDCl₃/TMS): 7,66 (d, J = 8,8 Hz, H-6,7), 7,58 (d, J = 9,5 Hz, H-1), 7,17 (dd, J = 9,5, 2,8 Hz, H-2), 7,13 (d, J = 2,8 Hz, H-4), 6,85 (d, J = 8,8 Hz, H-6,7), 6,78 (q, J = 8,1 Hz, H-11), 4,98 (d, J = 6,1 Hz, H-17), 3,92 (s, -OCH3), 2,11 (s, -OOC-CH3), 2,09 (s, -OOC-CH₃ ), 1,46 (dd, J = 4,9, 3,2 Hz, 14, 15-CH₂-), 0,97 (s, H-18), 0,57 (ddd, J = 8,2, 4,9, 1,7 Hz, 14,15-CH₂-). MS (m/z): 394 (M⁺), 334, 274, 259.

### Beispiel 4

### 11α-Hydroxy-3-methoxy-14α,15α-methylen-estra-1,3,5(10),6,8-pentaen-17α-yl-acetat aus 11α-Hydroxy-3-methoxy-14α,15α-methylen-8α,9α-oxido-estra-1,3,5(10)-trien-17α-yl-acetat

Analog zu Beispiel 3 wird die 11-Hydroxyverbindung mit Diphosphortetraiodid behandelt, wobei man die Titelverbindung erhält.
¹H-NMR (CDCl₃ / TMS): 8,26 (d, J = 9,4 Hz, H-1), 7,62 (d, J = 8,3 Hz, H-6,7), 7,22 (dd, J = 9,4, 2,7 Hz, H-2), 7,12 (d, J = 2,7 Hz, H-4), 6,83 (d, J = 8,3 Hz, H-6,7), 5,68 (q, J = 7,7 Hz, H-11), 4,99 (d, J = 6,3 Hz, H-17), 3,92 (s, -OCH₃), 2,10 (s, -OOC-CH₃), 0,93 (s, H-18), 0,57 (ddd, J = 7,6, 4,8, 1,6 Hz, 14,15-CH₂-). MS (m /z): 370 (M⁺), 353, 310, 292, 277, 267.

## Patentansprüche

1. Equileninderivate der allgemeinen Formel I 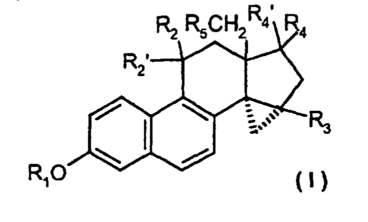 in der
R₁ ein Wasserstoffatom, eine C₁-C₅-Alkyl- oder eine C₁-C₅-Acylgruppe oder eine Benzoylgruppe bedeutet, R₂ ein Wasserstoffatom und R₂' ein Fluoratom, eine Hydroxygruppe oder eine C₁-C₅-Acyloxygruppe darstellt oder R₂ und R₂' zusammen eine Oxogruppe darstellen, R₃ ein Wasserstoffatom oder eine Methylgruppe darstellt, R₄ ein Wasserstoffatom und R₄' eine Hydroxygruppe oder eine C₁-C₁₁-Acyloxygruppe darstellt oder R₄ und R₄' zusammen eine Oxogruppe, eine Methylengruppe, eine Halogenmethylengruppe oder eine Dihalogenmethylengruppe darstellen und R₅ ein Wasserstoffatom oder eine Methylgruppe ist.

2. Equileninderivate gemäß Anspruch 1, **dadurch gekennzeichnet, daß** R₅ ein Wasserstoffatom ist.

3. Equileninderivate gemäß Anspruch 1, nämlich
1) 14α,15α-Methylen-estra-1,3,5(10),6,8-pentaen-3,11β,17β-triol,
2) 11β,17β-Dihydroxy-14α,15α-methylen-estra-1,3,5(10),6,8-pentaen-3-yl-benzoat,
3) 11β,17β-Dihydroxy-14α,15α-methylen-estra-1,3,5(10),6,8-pentaen-3-yl-propionat,
4) 3,11β-Dihydroxy-14α,15α -methylen-estra-1,3,5(10),6,8-pentaen-17β-yl-decanoat,
5) 3,11β-Dihydroxy-14α,15α-methylen-estra-1,3,5(10),6,8-pentaen-17-on,
6) 3-Methoxy-14α,15α-methylen-estra-1,3,5(10),6,8-pentaen-11α ,17β-diyl-diacetat,
7) 15β-Methyl-14α,15α-methylen-estra-1,3,5(10),6,8-pentaen-3,11β,17β-triol,
8) 11β-Fluor-14α,15α-methylen-estra-1,3,5(10),6,8-pentaen-3,17β-diol,
9) 3,17β-Dihydroxy-14α,15α-methylen-1,3,5(10),6,8-pentaen-11-on,
10) 3-Methoxy-14α,15α-methylen-estra-1,3,5(10),6,8-pentaen-11α,17α-diyl-diacetat,
11) 3-Methoxy-14α,15α-methylen-11-oxo-estra-1,3,5(10),6,8-pentaen-17α-yl-acetat,
12) 11 β-Hydroxy-17,17-difluormethylen-14α,15α -methylen-estra-1,3,5(10),6,8-pentaen-3-yl-benzoat und
13) 14α,15α,17,17-Bis-methylen-estra-1,3,5(10),6,8-pentaen-3,11α-diol.

4. Verfahren zur Herstellung der erfindungsgemäßen Equileninderivate der allgemeinen Formel I 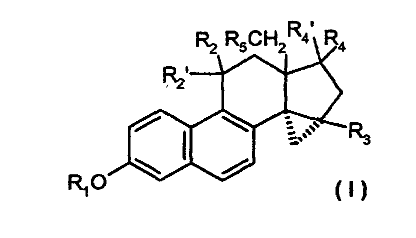 worin R₁, R₂, R₂', R₃, R₄, R₄' und R₅ die in Anspruch 1 gegebene Bedeutung haben, indem man eine Verbindung der allgemeinen Formel II 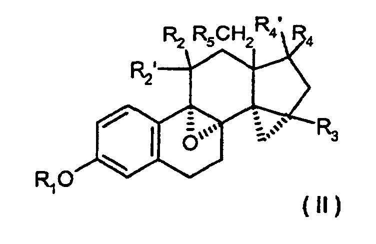 worin R₁, R₂, R₂', R₃, R₄, R₄' und R₅ die in Anspruch 1 gegebene Bedeutung haben, mit Diphosphortetraiodid in Gegenwart von Pyridin zur Reaktion bringt und die so erhaltenen Verbindungen in an sich bekannter Weise zu den Verbindungen der allgemeinen Formel I umsetzt.

5. Pharmazeutische Zusammensetzung, die mindestens eine Verbindung der allgemeinen Formel I nach den Ansprüchen 1 bis 3, gegebenenfalls zusammen mit pharmazeutisch verträglichen Hilfs- und Trägerstoffen enthält.

6. Verwendung der Verbindungen der allgemeinen Formel I nach den Ansprüchen 1 bis 3 zur Herstellung eines Arzneimittels zur Geroprophylaxe bei Mann und Frau.

7. Verbindungen der allgemeinen Formel I nach den Ansprüchen 1 bis 3 zur Anwendung als therapeutische Wirkstoffe.

8. Cyclopropano-Steroide der allgemeinen Formel II 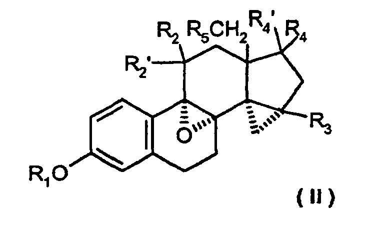 worin R₁, R₂, R₂', R₃, R₄, R₄' und R₅ die in Anspruch 1 gegebene Bedeutung haben.

9. Cyclopropano-Steroide gemäß Anspruch 8, nämlich
1. 11α-Hydroxy-3-methoxy-14α,15α-methylen-8α,9α-oxido-estra-1,3,5(10)-trien-17α-yl-acetat,
2. 3-Methoxy-14α,15α-methylen-8α,9α-oxido-estra-1,3,5(10)-trien-11α,17α-diyl-diacetat und
3. 3-Methoxy-11α-hydroxy-8α,9α-oxido-14α,15α-methylen-estra-1,3,5(10)-trien-17β-yl-acetat.

## Claims

1. Equilenin derivatives of the general formula I 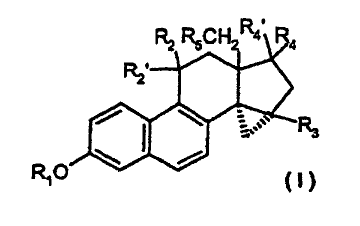 in which
R₁ is a hydrogen atom, a C₁-C₅-alkyl or a C₁-C₅-acyl group or a benzoyl group, R₂ is a hydrogen atom and R₂' is a fluorine atom, a hydroxyl group or a C₁-C₅-acyloxy group or R₂ and R₂' together are an oxo group, R₃ is a hydrogen atom or a methyl group, R₄ is a hydrogen atom and R₄' is a hydroxyl group or a C₁-C₁₁-acyloxy group or R₄ and R₄' together are an oxo group, a methylene group, a halomethylene group or a dihalomethylene group and R₅ is a hydrogen atom or a methyl group.

2. Equilenin derivatives according to Claim 1, **characterized in that** R₅ is a hydrogen atom.

3. Equilenin derivatives according to Claim 1, namely
1) 14α,15α-methyleneoestra-1,3,5(10),6,8-pentaene-3,11β,17β-triol,
2) 11β,17β-dihydroxy-14α,15α-methyleneoestra-1,3,5(10),6,8-pentaen-3-yl benzoate,
3) 11β,17β-dihydroxy-14α,15α-methyleneoestra-1,3,5(10),6,8-pentaen-3-yl propionate,
4) 3,11β-dihydroxy-14α,15α-methyleneoestra-1,3,5(10),6,8-pentaen-17β-yl decanoate,
5) 3,11β-dihydroxy-14α,15α-methyleneoestra-1,3,5(10),6,8-pentaen-17-one,
6) 3-methoxy-14α,15α-methyleneoestra-1,3,5(10),6,8-pentaene-11α,17β-diyl diacetate
7) 15β-methyl-14α,15α-methyleneoestra-1,3,5(10),6,8-pentaene-3,11β,17β-triol,
8) 11β-fluoro-14α,15α-methyleneoestra-1,3,5(10),6,8-pentaene-3,17β-diol,
9) 3,17β-dihydroxy-14α,15α-methyleneoestra-1,3,5(10),6,8-pentaen-11-one,
10) 3-methoxy-14α,15α-methyleneoestra-1,3,5(10),6,8-pentaene-11α,17α-diyl diacetate,
11) 3-methoxy-14α,15α-methylene-11-oxooestra-1,3,5(10),6,8-pentaen-17α-yl acetate,
12) 11β-hydroxy-17,17-difluoromethylene-14α,15α-methyleneoestra-1,3,5(10),6,8-pentaen-3-yl benzoate and 14α,15α,17,17-bis-methyleneoestra-1,3,5(10),6,8-pentaene-3,11α-diol.

4. Process for the preparation of the equilenin derivatives of the general formula I according to the invention 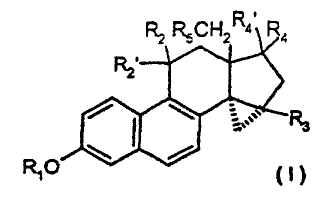 in which R₁, R₂, R₂', R₃, R₄, R₄' and R₅ have the meaning given in Claim 1,
by reacting a compound of the general formula II 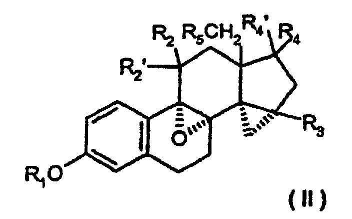 in which R₁, R₂, R₂', R₃, R₄, R₄' and R₅ have the meaning given in Claim 1, with diphosphorus tetraiodide in the presence of pyridine and reacting the compounds thus obtained in a manner known per se to give the compounds of the general formula I.

5. Pharmaceutical composition which contains at least one compound of the general formula I according to Claims 1 to 3, optionally together with pharmaceutically tolerable excipients and vehicles.

6. Use of the compounds of the general formula I according to Claims 1 to 3 for the production of a medicament for geroprophylaxis in men and women.

7. Compounds of the general formula I according to Claims 1 to 3 for use as therapeutic active compounds.

8. Cyclopropanosteroids of the general formula II 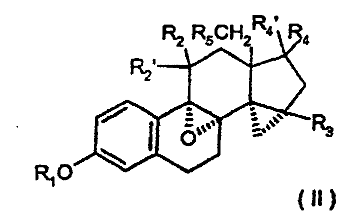 in which R₁, R₂, R₂', R₃, R₄, R₄' and R₅ have the meaning given in Claim 1.

9. Cyclopropanosteroids according to Claim 8, namely
1. 11α-hydroxy-3-methoxy-14α,15α-methylene-8α,9α-oxidooestra-1,3,5(10)-trien-17α-yl acetate,
2. 3-methoxy-14α,15α-methylene-8α,9α-oxidooestra-1,3,5(10)triene-11α,17α-diyl diacetate and
3. 3-methoxy-11α-hydroxy-8α,9α-oxido-14α,15α-methyleneoestra-1,3,5(10)-trien-17β-yl acetate.

## Revendications

1. Dérivés d'équilénine de formule générale I 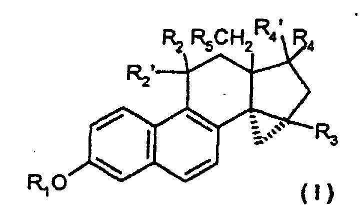 dans laquelle
R₁ représente un atome d'hydrogène, un groupe alkyle en C₁-C₅ ou acyle en C₁-C₅ ou un groupe benzoyle, R₂ représente un atome d'hydrogène et R₂' représente un atome de fluor, un groupe hydroxy ou un groupe acyloxy en C₁-C₅, ou R₂ et R₂' représentent conjointement un groupe oxo, R₃ représente un atome d'hydrogène ou un groupe méthyle, R₄ représente un atome d'hydrogène et R₄' représente un groupe hydroxy ou un groupe acyloxy en C₁-C₁₁, ou R₄ et R₄' représentent conjointement un groupe oxo, un groupe méthylène, un groupe halogénométhylène ou un groupe dihalogénométhylène, et R₅ représente un atome d'hydrogène ou un groupe méthyle.

2. Dérivés d'équilénine selon la revendication 1, **caractérisés en ce que** R₅ représente un atome d'hydrogène.

3. Dérivés d'équilénine selon la revendication 1, à savoir
1) le 14α,15α-méthylène-estra-1,3,5(10),6,8-pentaène-3,11β,17β-triol,
2) le 11β,17β-dihydroxy-14α,15α-méthylène-estra-1,3,5(10),6,8-pentaén-3-yl-benzoate,
3) le 11β,17β-dihydroxy-14α,15α-méthylène-estra-1,3,5(10),6,8-pentaén-3-yl-propionate,
4) le 3,11β-dihydroxy-14α,15α-méthylène-estra-1,3,5(10),6,8-pentaén-17β-yl-décanoate,
5) la 3,11β-dihydroxy-14α,15α-méthylène-estra-1,3,5(10),6,8-pentaén-17-one,
6) le 3-méthoxy-14α,15α-méthylène-estra-1,3,5(10),6,8-pentaène-11α,17β-diyl-diacétate,
7) le 15β-méthyl-14α,15α-méthylène-estra-1,3,5(10),6,8,-pentaène-3,11β,17β-triol,
8) le 11β-fluor-14α,15α-méthylène-estra-1,3,5(10),6,8,-pentaène-3,17β-diol,
9) la 3,17β-dihydroxy-14α,15α-méthylène-1,3,5(10),6,8,-pentaén-11-one,
10) le 3-méthoxy-14α,15α-méthylène-estra-1,3,5(10),6,8-pentaène-11α,17α-diyl-diacétate,
11) le 3-méthoxy-14α,15α-méthylène-11-oxo-estra-1,3,5(10),6,8-pentaén-17α-yl-acétate,
12) le 11β-hydroxy-17,17-difluorométhylène-14α,15α-méthylène-estra-1,3,5(10),6,8-pentaén-3-yl-benzoate et
13) le 14α,15α,17,17-bis-méthylène-estra-1,3,5(10),6,8-pentaène-3,11α-diol.

4. Procédé de préparation des dérivés d'équilénine selon l'invention de formule générale I 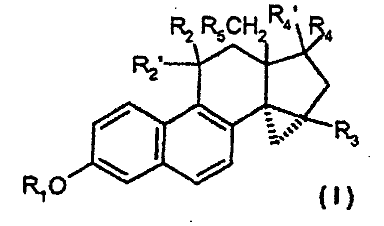 dans laquelle R₁, R₂, R_{2'}, R₃, R₄, R_{4'} et R₅ présentent la signification indiquée dans la revendication 1,
en faisant réagir un composé de formule générale II 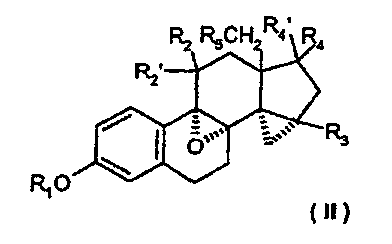 dans laquelle R₁, R₂, R_{2'}, R₃, R₄, R_{4'} et R₅ présentent la signification indiquée dans la revendication 1, avec du tétra-iodure de diphosphore en présence de pyridine, et en transformant les composés ainsi obtenus, de façon connue en soi, en les composés de formule générale I.

5. Composition pharmaceutique comprenant au moins un composé de formule générale I selon les revendications 1 à 3, éventuellement conjointement avec des auxiliaires et des supports pharmaceutiquement acceptables.

6. Utilisation des composés de formule générale I selon les revendications 1 à 3, pour la préparation d'un médicament destiné à la gérontoprophylaxie chez l'homme et la femme.

7. Composés de formule générale I selon les revendications 1 à 3, destinés à être utilisés en tant qu'agents actifs thérapeutiques.

8. Cyclopropano-stéroïdes de formule générale II 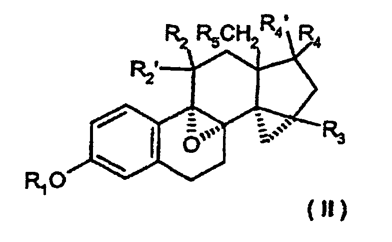 dans laquelle R₁, R₂, R_{2'}, R₃, R₄, R_{4'} et R₅ présentent la signification indiquée dans la revendication 1.

9. Cyclopropano-stéroïdes selon la revendication 8, à savoir
1. le 11α-hydroxy-3-méthoxy-14α,15α-méthylène-8α,9α-oxydo-estra-1,3,5(10)-trién-17α-yl-acétate,
2. le 3-méthoxy-14α,15α-méthylène-8α-9α-oxydo-estra-1,3,5(10)-triène-11α,17α-diyl-diacétate et
3. le 3-méthoxy-11α-hydroxy-8α-9α-oxydo-14α,15α-méthylène-estra-1,3,5(10)-trién-17β-yl-acétate.
